# EUROPEAN PATENT SPECIFICATION

(11) **EP 0 459 744 B1**
(45) Date of publication and mention of the grant of the patent: **09.10.1996**
(21) Application number: 91304789.0
(22) Date of filing: 28.05.1991
(51) Int. Cl.: C12P 7/40, A23L 1/03

(54) **Method of producing a fatty acid**
Verfahren zur Herstellung von Fettsäure
Méthode de production d'un acide gras

(30) Priority: 26.05.1990 GB 9011874
(43) Date of publication of application: 04.12.1991
(73) Proprietor: THE SCOTTISH AGRICULTURAL COLLEGE, Perth, PH1 1HF (GB)
(72) Inventor: Fallowfield, Howard John, Patna, Ayr KA6 7EZ (GB)
(74) Representative: Pattullo, Norman

(56) References cited:
- EP-A- 0 222 169
- EP-A- 0 355 972
- AQUACULTURE, vol. 97, no. 1, 1991; ASSAF SUKENIK et al., pp. 61-72
- MARINE BIOLOGY, vol. 95, no. 1, 1987; A. BEN-AMOTZ et al.; pp. 31-36

## Description

This invention relates to a method of producing a fatty acid in treating the body.

Fatty acids, particularly polyunsaturated fatty acids, play a major role in the functioning of human tissues and maintaining well being. In particular, docosahexaenoic acid (DHA) has been identified as having a major role in the prevention of many of the ailments which afflict modern society eg ischaemic heart disease, rheumatoid arthritis and associated conditions and the degeneration of tissue function.

The specific role of DHA has been identified and it is accepted that dietary supplementation with DHA is desirable. Presently fish oils are the predominant source of DHA for dietary supplement purposes.

It has also been suggested to extract DHA from young cow brains, but as only very small amounts of DHA are obtainable from cow brains, the economic value of cows as providers of meat and milk outweighs the benefit to be derived from killing them early in life to obtain DHA.

The present invention identifies an alternative source of DHA. The lipid product from this source can provide a very high level of DHA, exceeding that which is found in fish oil extracts, in a fatty acid combination that is particularly beneficial to the human condition.

Ben-Amotz (1987) in Marine Biology 97(1):31-37 discloses a comparative study to determine the best algal foodsource on which to feed rotifers for subsequent feeding to fish larvae.

EP 0,222,169A (The Nishin Oil Mills Ltd) discloses a feedstuff for cultivating Artemia as a feedstuff for fish larvae, the feedstuff comprising a product obtained by treatment of algal cell walls.

According to the present invention there is provided a method of producing DHA comprising treating a species of algae Isochrysis with nitrogen at a temperature of under 40°C and over 13°C, wherein the nitrogen is provided at a concentration of between 10mg and 50mg per litre.

The fatty acid profile of Isochrysis organisms is important for nutrition. Fish oil contains DHA but also eicosapentaenoic acid (EPA). EPA is a blood anti-agglutination factor and in some circumstances can cause problems when present in nutritional supplements. Isochrysis lipid contains DHA but no EPA; however, stearidonic acid, also present in the lipid fraction of Isochrysis, is a precursor from which EPA can be produced.

The DHA may be extracted from the treated algal species for use in introduction into human or animal bodies.

Preferably, the DHA is extracted from the treated algal species by solvent extraction.

The nitrogen may be provided in any suitable form, for example by using ammonia or a nitrate.

Preferably, the nitrate concentration in nitrate nitrogen per litre (NO₃-N/l), is between 12mg and 30mg NO₃-N/l; most preferably the concentration of nitrogen is 25mg NO₃-N/l.

Preferably, the temperature is between 14°C and 35°C. More preferably, the temperature is between 15°C and 30°C. Even more preferably the temperature is between 16°C and 25°C. Most preferably the temperature is 17°.

The algal species may be for example Isochrysis galbana or Isochrysis (Cambridge Collection of Algae and Protozoa No 927/14).

A description of the conditions of culture and results in embodiments of the invention will now be given by way of example, referring to the drawings in which:
Fig. 1 shows the changes in percentage concentration of DHA in Isochrysis lipid, produced under varying conditions;
Fig. 2 shows the changes in percentage concentration of stearidonic acid in Isochrysis lipid, produced under varying conditions;
Fig. 3 shows the concentration of DHA in Isochrysis as mg/100g dry weight, produced under varying conditions;
Fig. 4 shows the concentration of stearidonic acid in Isochrysis, as mg/100g dry weight; and
Fig. 5 is a table of data collected by culturing samples of Isochrysis at differing temperatures and nitrogen concentrations.

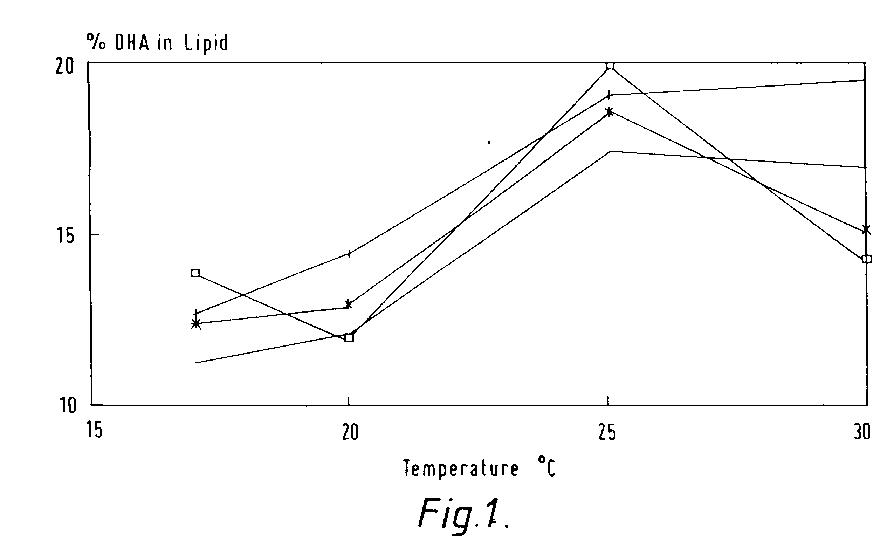

The key for Figs. 1 to 4 is as follows:
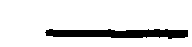 12.7mg NO₃-N (E)
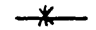 25.0mg NO₃-N (E)
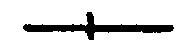 12.7mg NO₃-N (S)
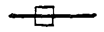 25.0mg NO₃-N (S)

Stearidonic acid is denoted in fatty acid nomenclature as 18:4 (n-3), abbreviated to 18:4 in this specification.

The fatty acid profile of Isochrysis differs during rapid growth (exponential growth phase) and a more quiescent period (stationary growth phase) typically occurring when nutrients are not abundantly available.

### EXPERIMENTAL DETAILS AND RESULTS

### Culture Conditions

- Growth Media :: (g/l) Na₂EDTA, 0.00436; FeCl₃.6H₂O, 0.00315;
CuSO₄.5H₂O, 0.0001; CoCl₂.6H₂O
0.00001; MnCl₂.4H₂O
0.00018; Na₂MoO₄.2H₂O,
0.000006; ZnSO₄.7H₂O,
0.000022; NaH₂PO₄.2H₂O,
0.00565;
- : (ug/l) Cyanocobalamin, 0.05;
Thiamine, 100; Biotin, 0.5.
Made up to one litre with artificial seawater (33.6g
Ultramarine Synthetica Seasalt per litre distilled water).
- Nitrogen :: 25mg NO₃-N/1
- pH :: 8.0
- Culture Irradiance :: 100umoles/m²/s photosynthetically active radiation (PAR 400-700nm).
- Culture Temperature :: 17°C, thermostatically controlled water bath.
- Culture Air Supply :: 500ml/min.

The product, obtained from the above conditions contains the following fatty acids; 17.5% docosahexaenoic acid (DHA), 5% octadecatrienoic acid, 3.1% octadecadienoic acid, 14.5% octadecatetraenoic acid, 12% hexadecanoic acid and 18.9% tetradecanoic acid.

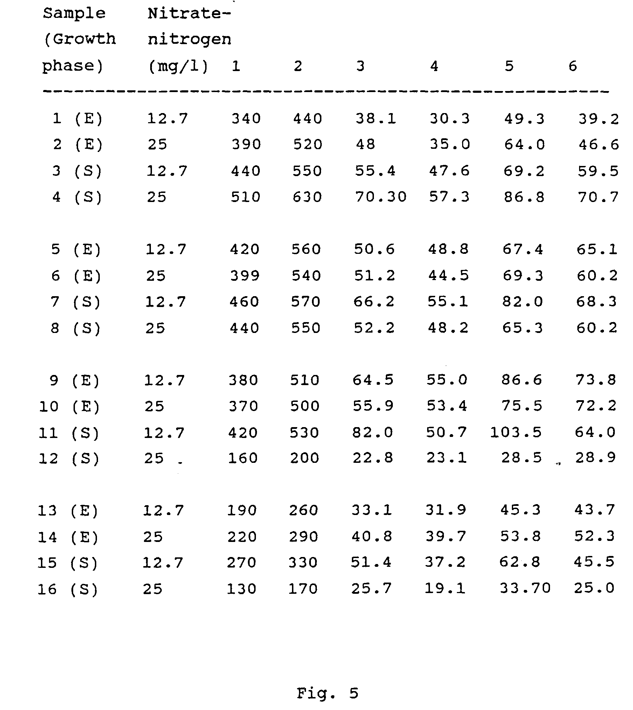

In further experiments, the nitrogen and temperatures used were varied (see Fig. 5).

### Fatty Acid Preparation and Identification

Fatty acid content was determined as their methyl esters. The esters were prepared by the direct methylation of the freeze dried cell material by boiling with KOH-MeOH overnight, or by boiling with H₂SO₄-MeOH overnight.

Esters were extracted into heptane, following the addition of saturated salt solution. The heptane extract was dried to a suitable volume for gas liquid chromatographic analysis (g.l.c.).

Fatty acid gas-liquid chromatography - heptane extracts were passed through a Fluorosil pre-column and separated using a WCOT fused silica CP-Sil 88 (50m - 0.25 internal diameter) capillary column with a 5m deactivated fused silica pre-column. Conditions: maximum column temperature 226°C, detector 300°C, injector 240°C. Oven temperature, initial 160°C - final 225°C - rise 5°C/min. Helium carrier gas 125 Kpa; split ratio 100:1; flow 0.43 ml/min.

The starting concentration of DHA in Isochrysis is approximately 8% of total fatty acid content.

The results of varying the temperature and concentrations can be seen in graphic form in Figs. 1 - 3, which relate these variations to the DHA and 18:4 content. The exponential and stationary growth phases are denoted as E and S respectively in Fig. 5 and are measurements after 14(E) and 21(S) days of treatment, Columns 1 - 6 (Fig 5) are annotated as follows:
- Column 1 -: lipid concentration
- Column 2 -: conversion factor applied
- Column 3 -: DHA
- Column 4 -: 18:4
- Column 5 -: DHA (based on corrected lipid factor, Column 2)
- Column 6: - 18:4 (based on corrected lipid factor, Column 2)

The temperatures for each experiment are as follows:

| Rows | °C |
|---|---|
| 1 - 4 | 17 |
| 5 - 8 | 20 |
| 9 - 12 | 30 |
| 13 - 16 | 25 |

Units for data in Fig. 5 are mg/100g dry weight.

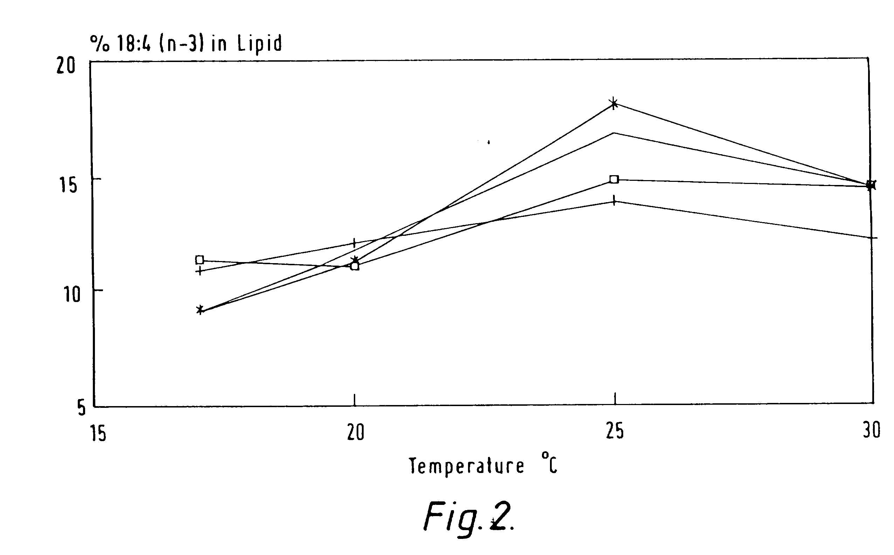

Figs 1 and 2 show the percentage of DHA or 18:4, present under different culture conditions of temperature and nitrogen concentration.

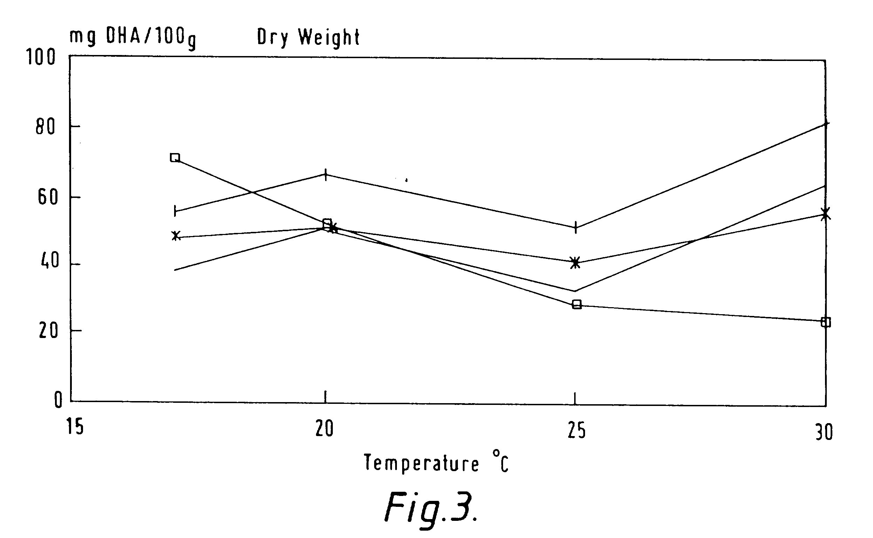

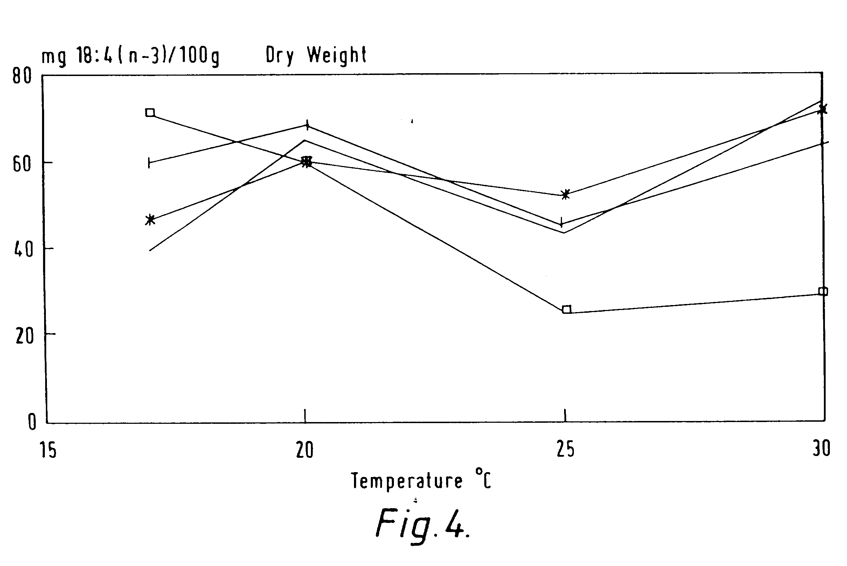

The total yield of each fatty acid from dry weight is shown in Figs 3 and 4. Comparison of the Figures shows that despite the maximum concentration of each fatty acid, being obtained at 25°C and 25mg nitrate-nitrogen/litre, the best dry yield is obtained by culture conditions of 30°C and 12.7mg nitrate-nitrogen/litre (stationary phase) which could be more desirab1e commercially. High production of DHA is also achieved in the conditions outlined in Row 4 - 25mg nitrate-nitrogen per litre at 17°C, which is detailed in the experimental conditions outlined above. The conditions chosen may thus be tailored to suit the requirements of the user.

Fig. 5 details percentage lipid and absolute amounts of DHA and 18:4 found in Isochrysis 927/14. The total lipid is determined from the sum of the fatty acids measured following methylation and glc. However, lipid fractions eg phospholipid have other moieties associated with them-and so recognised conversion factors are applied to calculate the 'true' lipid content (Column 2). The DHA and 18:4 percentages of importance are shown in Columns 5 and 6.

Inspection of the data for I galbana also shows relatively high levels of DHA and 18:4.
I galbana 17°C 25 mg NO₃-N
Exponential growth phase DHA 10.8%; 18:4 6.2%
Stationary growth phase DHA 15.5%; 18.4 7.6%

Modifications and improvements may be incorporated without departing from the scope of the invention.

## Claims

1. A method of producing DHA comprising treating a species of algae Isochrysis with nitrogen at a temperature of under 40°C and over 13°C, wherein the nitrogen is provided at a concentration between 10mg and 50mg per litre.

2. A method of producing DHA as claimed in Claim 1, wherein the DHA is subsequently extracted from the treated algal species.

3. A method of producing DHA as claimed in Claim 1 or 2 wherein the nitrogen is in the form of a nitrate or ammonia.

4. A method of producing DHA as claimed in any preceding Claim, wherein the nitrogen is provided at a concentration between 12mg and 30mg per litre.

5. A method of producing DHA as claimed in any preceding Claim, wherein the concentration of nitrogen is 25mg per litre.

6. A method of producing DHA as claimed in any preceding Claim, wherein the temperature is between 14°C and 35°C.

7. A method of producing DHA as claimed in Claim 6 wherein the temperature is between 15°C and 30°C.

8. A method of producing DHA as claimed in Claim 7 wherein the temperature is between 16°C and 25°C.

9. A method of producing DHA as claimed in Claim 1, wherein the temperature is 30°C and the concentration of nitrogen is 12.7mg NO₃-N/l.

10. A method of producing DHA as claimed in Claim 1, wherein the temperature is 17°C and the concentration of nitrogen is 25mg NO₃-N/l.

11. A method of producing DHA as claimed in any preceding Claim, wherein the algae are maintained in the stationary growth phase.

## Patentansprüche

1. Verfahren zur Herstellung von DHA, bei welchem eine Spezies der Algen Isochrysis mit Stickstoff bei einer Temperatur von weniger als 40°C und mehr als 13°C behandelt wird, wobei der Stickstoff mit einer Konzentration zwischen 10 mg und 50 mg pro Liter bereitgestellt wird.

2. Verfahren zur Herstellung von DHA nach Anspruch 1, bei welchem DHA anschließend von der behandelten Algenspezies extrahiert wird.

3. Verfahren zur Herstellung von DHA nach Anspruch 1 oder 2, bei welchem der Stickstoff in der Form eines Nitrats oder von Ammoniak ist.

4. Verfahren zur Herstellung von DHA nach einem der vorhergehenden Ansprüche, bei welchem der Stickstoff mit einer Konzentration zwischen 12 mg und 30 mg pro Liter bereitgestellt wird.

5. Verfahren zur Herstellung von DHA nach einem der vorhergehenden Ansprüche, bei welchem die Konzentration von Stickstoff 25 mg pro Liter beträgt.

6. Verfahren zur Herstellung von DHA nach einem der vorhergehenden Ansprüche, bei welchem die Temperatur zwischen 14°C und 35°C beträgt.

7. Verfahren zur Herstellung von DHA nach Abspruch 6, bei welchem die Temperatur zwischen 15°C und 30°C beträgt.

8. Verfahren zur Herstellung von DHA nach Abspruch 7, bei welchem die Temperatur zwischen 16°C und 25°C beträgt.

9. Verfahren zur Herstellung von DHA nach Abspruch 1, bei welchem die Temperatur 30°C beträgt und die Konzentration von Stickstoff 12.7 mg NO₃-N/l beträgt.

10. Verfahren zur Herstellung von DHA nach Abspruch 1, bei welchem die Temperatur 17°C beträgt und die Konzentration von Stickstoff 25 mg NO₃-N/l beträgt.

11. Verfahren zur Herstellung von DHA nach einem der vorhergenden Absprüche, bei welchem die Algen in der stationären Wachstumsphase beibehalten werden.

## Revendications

1. Procédé de production de DHA comprenant le traitement d'une espèce d'algues isochrysis avec de l'azote à une température inférieure à 40 °C et supérieure à 13 °C, dans lequel l'azote est fourni à une concentration comprise entre 10 mg et 50 mg par litre.

2. Procédé de production de DHA selon la revendication 1, dans lequel le DHA est ensuite extrait de l'espèce d'algues traitée.

3. Procédé de production de DHA selon la revendication 1 ou la revendication 2 dans lequel l'azote est sous la forme d'un nitrate ou d'ammoniac.

4. Procédé de production de DHA selon l'une quelconque des revendications précédentes, dans lequel l'azote est fourni à une concentration comprise entre 12 mg et 30 mg par litre.

5. Procédé de production de DHA selon l'une quelconque des revendications précédentes, dans lequel la concentration d'azote est de 25 mg par litre.

6. Procédé de production de DHA selon l'une quelconque des revendications précédentes, dans lequel la température est comprise entre 14 °C et 35 °C.

7. Procédé de production de DHA selon la revendication 6 dans lequel la température est comprise entre 15 °C et 30 °C.

8. Procédé de production de DHA selon la revendication 7, dans lequel la température est comprise entre 16 °C et 25 °C.

9. Procédé de production de DHA selon la revendication 1, dans lequel la température est de 30 °C et la concentration en azote est de 12.7 mg de NO₃-N/l.

10. Procédé de production de DHA selon la revendication 1, dans lequel la température est de 17 °C et la concentration d'azote est de 25 mg de NO₃-N/l.

11. Procédé de production de DHA selon l'une quelconque des revendications précédentes, dans lequel les algues sont maintenues dans la phase de croissance stationnaire.
